# EUROPEAN PATENT APPLICATION

(11) **EP 4 212 611 A1**
(43) Date of publication of application: **19.07.2023**
(21) Application number: 21877355.4
(22) Date of filing: 21.09.2021
(51) Int. Cl.: C12M 3/00, B01J 19/00, B81B 1/00, C12Q 1/02, G01N 37/00

(54) **MICROFLUIDIC DEVICE AND METHOD FOR USING MICROFLUIDIC DEVICE**

(30) Priority: 07.10.2020 JP 2020170005
(71) Applicant: Ushio Denki Kabushiki Kaisha, Tokyo 100-8150 (JP)
(72) Inventor: TAKAHASHI,Seiichiro, Tokyo 100-8150 (JP)
(74) Representative: Tomerius, Isabel
(86) International application number: PCT/JP2021/034538
(87) International publication number: WO 2022/075054

(57) **Abstract**

Disclosed herein are a microfluidic device that has a simple structure and is capable of performing perfusion culture and forming a cell layer barrier keeping a uniform cell density and a method for using such a microfluidic device. A microfluidic device 1 includes a first substrate 10, a second substrate 20 partially bonded to the first substrate 10, a first channel 25, a second channel 26, and a third channel 27 which extend between the first substrate 10 and the second substrate 20, first channel ports 21 and 22 connected to the first channel 25, at least one second channel port 23 connected to the second channel 26, and at least one third channel port 24 connected to the third channel 27, wherein a principal surface 20b-side wall surface 25d of the first channel 25 is closer to a principal surface 20b than a principal surface 20b-side wall surface 26d of the second channel 26 and a principal surface 20b-side wall surface 27d of the third channel 27.

## Description

### TECHNICAL FIELD

The present invention relates to a microfluidic device and a method for using the microfluidic device.

### BACKGROUND ART

The epithelial (endothelial) cell layer is formed by binding between cells and binding between cells and the extracellular matrix (ECM). This cell layer functions not only as the boundary of epithelial (endothelial) tissue but also as a barrier to actively limit permeation of substances. In a living body, this barrier is present in, for example, the small-intestinal mucosa, the respiratory tract mucosa, the blood vessels, the blood-brain barrier (BBB), the cornea, and the epidermis.

It is an important subject in drug discovery to know the response, such as permeation (absorption), toxicity, and metabolism, of a substance to be studied to the barrier, and therefore in-vitro assay systems using cell culture have been developed. In such assay systems, both surfaces of a cell layer barrier need to be separated by a compartment. Therefore, such culture vessels having a compartment separated by an artificial support as will be described below have been developed.

Patent Document 1 that will be mentioned below discloses technology related to a cell culture chamber and a tissue model using the cell culture chamber and a method for producing the tissue model.

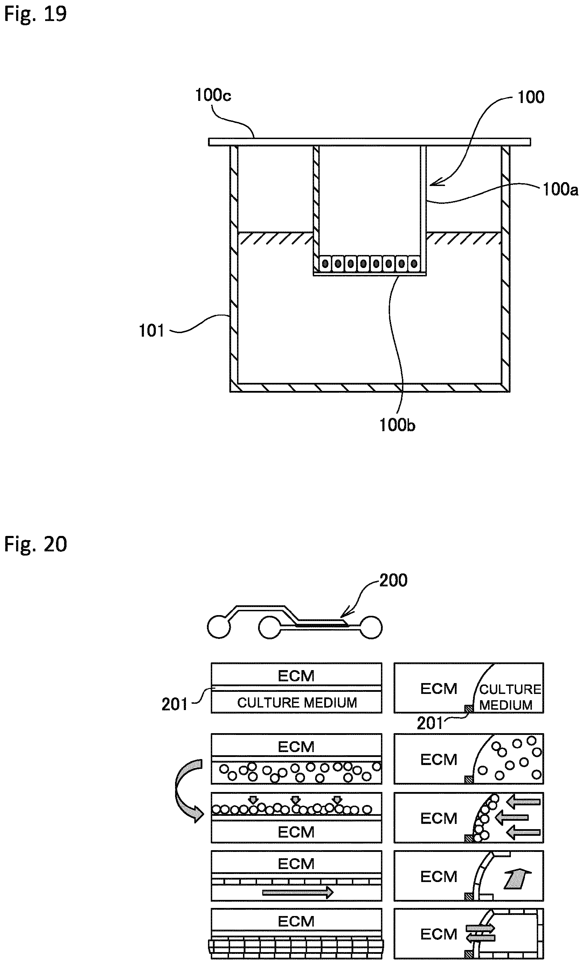

Fig. 19 is a schematic sectional view showing a state where a cell culture chamber 100 disclosed in Patent Document 1 is inserted into a container 101. A cylindrical frame body 100a, made of acrylic or polystyrene, has a space for holding cells therein. The cell culture chamber 100 has a dried Vitrigel membrane 100b fixed to cover one open end of the cylindrical frame body 100a and a locking part 100c provided at the outer periphery of the other open end of the cylindrical frame body 100a so as to project outwardly. The cell culture chamber 100 is inserted from the top of the container 101 so that the locking part 100c is hung on the top of the container 101 and the cell culture chamber 100 is held in the container 101. A physiological active substance can be injected into the container 101, and therefore the influence of the physiological active substance on cells can be assayed by allowing the physiological active substance to penetrate into cultured cells through the Vitrigel membrane.

Further, Patent Document 2 discloses a technique about a microfluidic plate capable of providing data of response of a compound to epithelial barrier function.

Fig. 20 is a schematic diagram showing the step of forming a tubule with the use of a microfluidic plate disclosed in Patent Document 2. The figure on the left in Fig. 20 is a partially enlarged view of the microfluidic plate, and the figure on the right in Fig. 20 is a sectional view of the microfluidic plate. The microfluidic plate has an entrance, a microfluidic chamber 200 connected to the entrance, and a projection, called a phase guide 201, provided in a longitudinal direction. A first fluid (e.g., ECM shown in Fig. 20) flows from the entrance to the microfluidic chamber 200 so that the microfluidic chamber 200 is selectively filled with the first fluid by the action of, for example, capillary force and surface tension by the phase guide 201, and gravity. The remaining portion is filled with a second fluid containing cells (e.g., a culture medium shown in Fig. 20) to form a tubule by cell culture.

### PRIOR ART DOCUMENTS

### PATENT DOCUMENTS

Patent Document 1: JP-A-2012-115262
Patent Document 2: JP-T-2018-522586

### SUMMARY OF THE INVENTION

### PROBLEMS TO BE SOLVED BY THE INVENTION

However, in the case of the structure disclosed in Patent Document 1, it is necessary to separately prepare the cell culture chamber 100 covered with a gel membrane in addition to the container 101, and as shown in Fig. 1 in Patent Document 1, many production steps are required to produce such a cell culture chamber 100.

Further, an artificial support is present under the gel membrane, which limits permeation of cells and substances which actually occurs in a living body.

Further, due to the structure of the cell culture chamber 100, a culture medium is not allowed to flow, and therefore only static culture can be performed. That is, perfusion culture cannot be performed. Therefore, the shear stress of a fluid and the partial pressure of a gas, which have influence on the expression of cell function, cannot be controlled.

The structure disclosed in Patent Document 2 is a mechanism utilizing capillary force and surface tension by the phase guide 201, and therefore the first fluid (e.g., ECM shown in Fig. 20) has a curved surface. Therefore, a cell layer barrier cultured on such a surface also has a curved surface, which makes it difficult to produce and observe the cell layer barrier.

Further, cells not in contact with the extracellular matrix (ECM) are also present, and therefore it is difficult to know the response of a subject to be studied only to the cell layer barrier due to a biased culture environment.

Further, the cell layer barrier is formed using surface tension, which limits the height of a channel. Therefore, the amount of a culture medium cannot be increased, and thus the culture medium needs to frequently be replaced.

In view of the above problems, it is an object of the present invention to provide a microfluidic device that has a simple structure and is capable of performing perfusion culture and forming a cell layer barrier keeping a uniform cell density and a method for using such a microfluidic device.

### MEANS FOR SOLVING THE PROBLEMS

The present invention is directed to a microfluidic device, including
a first substrate;
a second substrate partially bonded to the first substrate;
a first channel extending in a direction along a first principal surface of the second substrate between the first substrate and the second substrate;
a second channel connected to the first channel and extending in a direction along the first principal surface;
a third channel connected to the first channel and extending in a direction along the first principal surface;
a plurality of first channel ports connected to the first channel and formed to pass through the second substrate toward a second principal surface located opposite to the first principal surface of the second substrate;
at least one second channel port connected to the second channel and formed to pass through the second substrate toward the second principal surface; and
at least one third channel port connected to the third channel and formed to pass through the second substrate toward the second principal surface, wherein
a second principal surface-side wall surface of the first channel is closer to the second principal surface than a second principal surface-side wall surface of the second channel and a second principal surface-side wall surface of the third channel.

The microfluidic device has a simple structure that can be produced by previously forming the channels and the channel ports in the first substrate or the second substrate and then bonding the first substrate and the second substrate together. Further, for example, a culture medium or the like can be flowed from one of the first channel ports to another first channel port through the first channel. Further, for example, a culture medium or the like can also be flowed from the second channel port to the third channel port through the second channel, the first channel, and the third channel. That is, the microfluidic device according to the present invention is capable of performing perfusion culture.

The present invention is also directed to a method for using the microfluidic device, including:
a first step in which glycerol or thermal phase transition hydrogel is injected through the second channel port or the third channel port so that a height of the glycerol or the thermal phase transition hydrogel is higher than the second principal surface-side wall surface of the second channel and the second principal surface-side wall surface of the third channel and lower than the second principal surface-side wall surface of the first channel;
a second step in which an aqueous solution of gel is injected through one of the first channel ports;
a third step in which the aqueous solution of gel is gelatinized; and
a fourth step in which the glycerol or the thermal phase transition hydrogel is discharged through the third channel port or the second channel port.

This method of use is capable of forming a gel at a position higher than the second principal surface-side wall surface of the second channel and the second principal surface-side wall surface of the third channel and lower than the second principal surface-side wall surface of the first channel, and therefore a cell layer barrier keeping a uniform cell density can be formed on the gel.

### BRIEF DESCRIPTION OF THE DRAWINGS

Fig. 1 is a perspective view showing a state before a microfluidic device according to an embodiment of the present invention is produced.
Fig. 2 is a perspective view of a second substrate before bonding viewed from a first principal surface side.
Fig. 3 is a plan view of the microfluidic device.
Fig. 4 is a sectional view of the microfluidic device taken along line A-A shown in Fig. 3.
Fig. 5 shows sectional views of the microfluidic device respectively taken along line B-B and line C-C shown in Fig. 3.
Fig. 6A is a sectional view for describing Usage Example 1 of the microfluidic device.
Fig. 6B is a sectional view for describing Usage Example 1 of the microfluidic device.
Fig. 6C is a sectional view for describing Usage Example 1 of the microfluidic device.
Fig. 6D is a sectional view for describing Usage Example 1 of the microfluidic device.
Fig. 6E is a sectional view for describing Usage Example 1 of the microfluidic device.
Fig. 6F is a sectional view for describing Usage Example 1 of the microfluidic device.
Fig. 6G is a sectional view for describing Usage Example 1 of the microfluidic device.
Fig. 7A is a sectional view for describing Usage Example 2 of the microfluidic device.
Fig. 7B is a sectional view for describing Usage Example 2 of the microfluidic device.
Fig. 7C is a sectional view for describing Usage Example 2 of the microfluidic device.
Fig. 7D is a sectional view for describing Usage Example 2 of the microfluidic device.
Fig. 7E is a sectional view for describing Usage Example 2 of the microfluidic device.
Fig. 7F is a sectional view for describing Usage Example 2 of the microfluidic device.
Fig. 7G is a sectional view for describing Usage Example 2 of the microfluidic device.
Fig. 8A is a sectional view for describing Usage Example 3 of the microfluidic device.
Fig. 8B is a sectional view for describing Usage Example 3 of the microfluidic device.
Fig. 8C is a sectional view for describing Usage Example 3 of the microfluidic device.
Fig. 8D is a sectional view for describing Usage Example 3 of the microfluidic device.
Fig. 9A is a sectional view for describing Usage Example 4 of the microfluidic device.
Fig. 9B is a sectional view for describing Usage Example 4 of the microfluidic device.
Fig. 9C is a sectional view for describing Usage Example 4 of the microfluidic device.
Fig. 9D is a sectional view for describing Usage Example 4 of the microfluidic device.
Fig. 9E is a sectional view for describing Usage Example 4 of the microfluidic device.
Fig. 10A is a sectional view for describing Usage Example 5 of the microfluidic device.
Fig. 10B is a sectional view for describing Usage Example 5 of the microfluidic device.
Fig. 10C is a sectional view for describing Usage Example 5 of the microfluidic device.
Fig. 10D is a sectional view for describing Usage Example 5 of the microfluidic device.
Fig. 10E is a sectional view for describing Usage Example 5 of the microfluidic device.
Fig. 11A is a sectional view for describing Usage Example 6 of the microfluidic device.
Fig. 11B is a sectional view for describing Usage Example 6 of the microfluidic device.
Fig. 11C is a sectional view for describing Usage Example 6 of the microfluidic device.
Fig. 12A is a sectional view for describing Usage Example 7 of the microfluidic device.
Fig. 12B is a sectional view for describing Usage Example 7 of the microfluidic device.
Fig. 12C is a sectional view for describing Usage Example 7 of the microfluidic device.
Fig. 12D is a sectional view for describing Usage Example 7 of the microfluidic device.
Fig. 12E is a sectional view for describing Usage Example 7 of the microfluidic device.
Fig. 13 shows plan and sectional views of a microfluidic device according to another embodiment of the present invention.
Fig. 14 shows plan and sectional views of a microfluidic device according to another embodiment of the present invention.
Fig. 15 shows plan and sectional views of a microfluidic device according to another embodiment of the present invention.
Fig. 16 shows plan and sectional views of a microfluidic device according to another embodiment of the present invention.
Fig. 17 is a plan view of a plate including the microfluidic devices.
Fig. 18 is a sectional view of a microfluidic device according to another embodiment of the present invention.
Fig. 19 is a schematic sectional view showing a state where a cell culture chamber disclosed in Patent Document 1 is inserted into a container.
Fig. 20 is a schematic diagram showing the step of forming a tubule with the use of a microfluidic plate disclosed in Patent Document 2.

### MODE FOR CARRYING OUT THE INVENTION

A microfluidic device according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

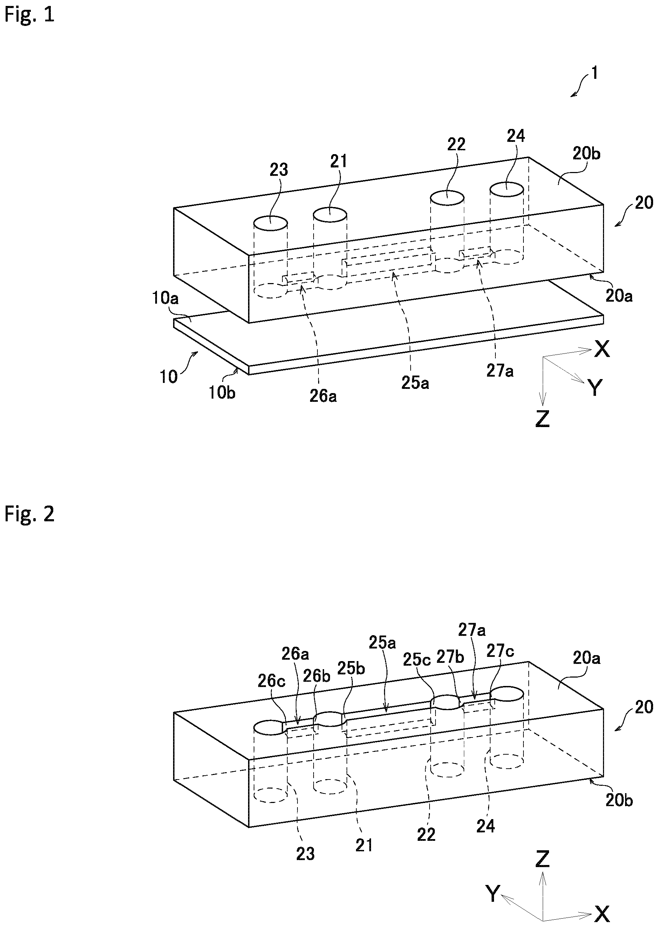

Fig. 1 is a perspective view showing a state before a microfluidic device 1 according to an embodiment of the present invention is produced. The microfluidic device 1 has a first substrate 10 and a second substrate 20 and is produced by bonding them together. Fig. 1 corresponds to a perspective view showing both of the first substrate 10 and the second substrate 20 just before bonding.

The microfluidic device 1 is formed by stacking the second substrate 20 on the first substrate 10 so that one principal surface 20a (corresponding to a first principal surface in the present invention) of the second substrate 20 is partially in contact with one principal surface 10a of the first substrate 10 and bonding them together. The principal surface refers to one of surfaces constituting the substrate 10 or 20 and having a much larger area than other surfaces. The substrate 10 or 20 has two principal surfaces, and these two principal surfaces are opposed to each other. The principal surface 20a of the second substrate 20 partially in contact with the first substrate 10 has recesses (which will be described later). The other principal surface 20b (corresponding to a second principal surface in the present invention) of the second substrate 20 is located on the opposite side from the first substrate 10 and has channel ports 21 to 24 (which will be described later).

In the following description, an XYZ coordinate system is appropriately referenced in which, in a state where the first substrate 10 and the second substrate 20 are bonded together, a plane parallel to the principal surfaces 10a and 10b of the first substrate 10 and the principal surfaces 20a and 20b of the second substrate 20 is defined as an XY plane and a direction orthogonal to the XY plane is defined as a Z direction.

When it is necessary to make a distinction between positive or negative to express a direction herein, the direction is described with a positive or negative sign, such as "+X direction" or "-X direction". When it is not necessary to make a distinction between positive or negative to express a direction, the direction is simply described as "X direction". Namely, when the direction is simply described as "X direction" herein, both "+X direction" and "-X direction" are included. The same applies to the Y direction and the Z direction. It should be noted that the microfluidic device 1 is usually used so that the Z direction corresponds to a vertical direction, and the -Z direction corresponds to an upward direction.

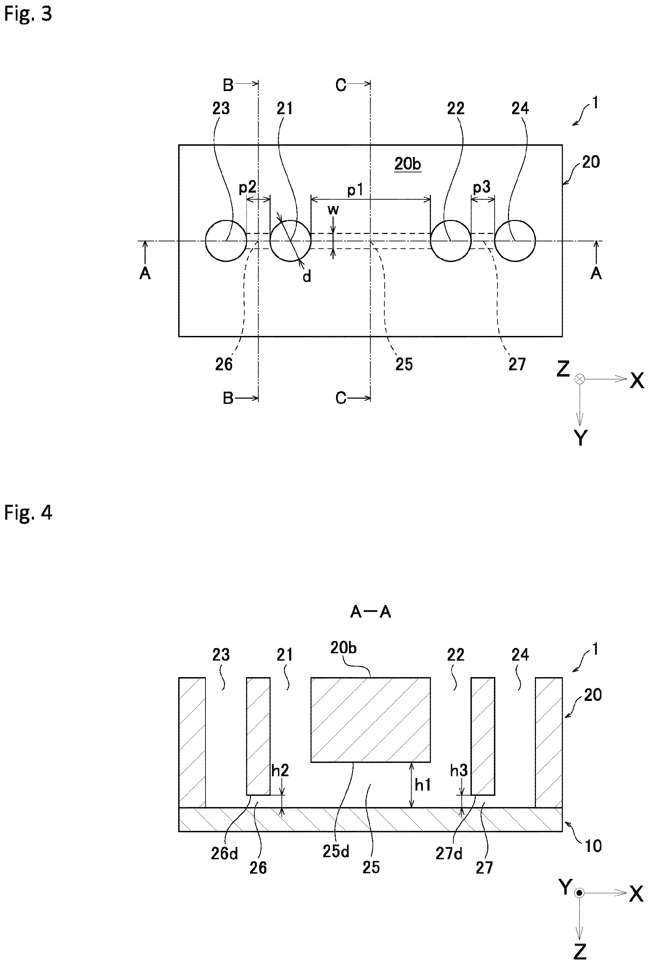

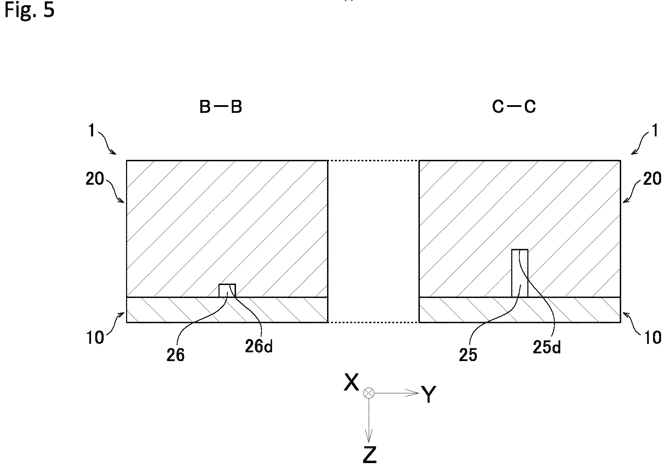

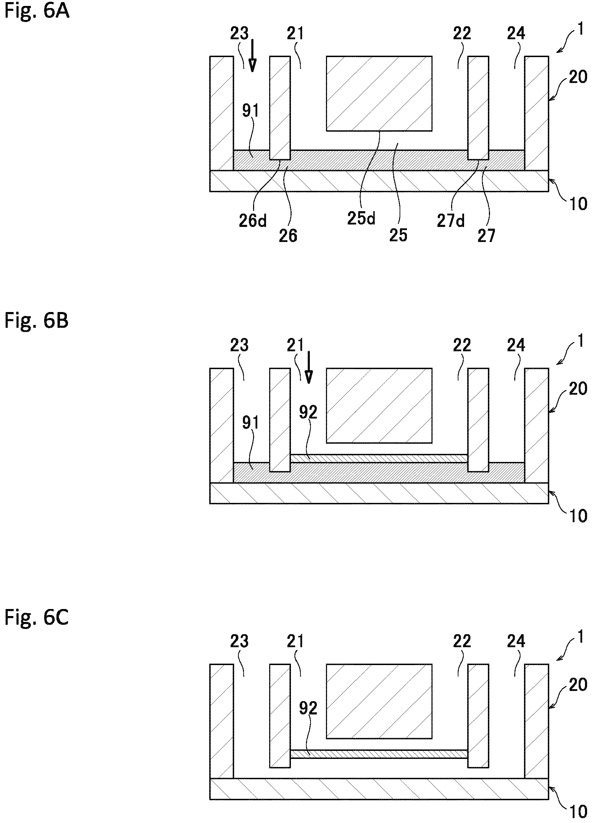

Fig. 2 is a perspective view of the second substrate 20 before bonding viewed from the principal surface 20a side. Fig. 3 is a plan view of the microfluidic device 1. Fig. 4 is a schematic sectional view of the microfluidic device 1 in which the second substrate 20 is bonded onto the first substrate 10, and this schematic sectional view is a sectional view of the microfluidic device 1 taken along line A-A shown in Fig. 3. In the schematic sectional view of Fig. 4, among outlines of both of the substrates, only lines appearing in the section are shown to facilitate understanding of the diagram. The same applies to Fig. 6A to Fig. 16 described later. Fig. 5 shows sectional views of the microfluidic device 1 respectively taken along line B-B and line C-C shown in Fig. 3.

The first substrate 10 and the second substrate 20 are each a rectangular substrate having principal surfaces that are the same in shape. The length and width of the principal surface of each of the first substrate 10 and the second substrate 20 are, for example, 15 mm and 25 mm, respectively. The thickness of the second substrate 20 is larger than that of the first substrate 10. The thickness of the first substrate 10 is, for example, 1 mm and the thickness of the second substrate 20 is, for example, 5 mm.

The principal surface 20a of the second substrate 20 includes a first recess 25a, a second recess 26a, and a third recess 27a. When the first substrate 10 and the second substrate 20 are bonded together, the first recess 25a, the second recess 26a, and the third recess 27a function as a hollow first channel 25, a hollow second channel 26, and a hollow third channel 27 sandwiched between both of the substrates 10 and 20.

The first recess 25a, the second recess 26a, and the third recess 27a are all formed in the center of the principal surface 20a in the Y direction to extend in the X direction. The first recess 25a, the second recess 26a, and the third recess 27a in this embodiment are arranged in a linear manner.

The first recess 25a, the second recess 26a, and the third recess 27a each have a slit shape extending in the X direction to have constant width and depth. The first recess 25a, the second recess 26a, and the third recess 27a are all the same in width w in the Y direction (see Fig. 3) and have a width w of, for example, 0.5 to 5 mm.

On the other hand, the first recess 25a, the second recess 26a, and the third recess 27a are different in depth from the principal surface 20a, and a depth h1 of the first recess 25a (see Fig. 4) is larger than a depth h2 (see Fig. 4) of the second recess 26a and a depth h3 (see Fig. 4) of the third recess 27a. Therefore, in a state where the second substrate 20 is bonded onto the first substrate 10, as shown in Fig. 4, a principal surface 20b-side wall surface 25d of the first channel 25 is closer to the principal surface 20b than a principal surface 20b-side wall surface 26d of the second channel 26 and a principal surface 20b-side wall surface 27d of the third channel 27. It should be noted that the depth h2 of the second recess 26a and the depth h3 of the third recess 27a may be the same or different, that is, the wall surface 26d of the second channel 26 and the wall surface 27d of the third channel 27 may be at the same position or different positions in the Z direction. The depth h1 of the first recess 25a is, for example, 2.5 to 5 mm and is 2.5 mm in this embodiment. The depth h2 of the second recess 26a and the depth h3 of the third recess 27a are, for example, 0.1 to 0.5 mm.

The first channel port 21 is connected to one end 25b of the first recess 25a and is formed to extend from the principal surface 20a of the second substrate 20 toward the principal surface 20b and to pass through the second substrate 20.

Further, the first channel port 21 is connected also to one end 26b of the second recess 26a. That is, the second recess 26a is connected to the first recess 25a through the first channel port 21.

The first channel port 22 is connected to the other end 25c of the first recess 25a and is formed to extend from the principal surface 20a of the second substrate 20 toward the principal surface 20b and to pass through the second substrate 20. Further, the first channel port 22 is connected also to one end 27b of the third recess 27a. That is, the third recess 27a is connected to the first recess 25a through the first channel port 22.

The second channel port 23 is connected to the other end 26c of the second recess 26a and is formed to extend from the principal surface 20a of the second substrate 20 toward the principal surface 20b and to pass through the second substrate 20.

The third channel port 24 is connected to the other end 27c of the third recess 27a and is formed to extend from the principal surface 20a of the second substrate 20 toward the principal surface 20b and to pass through the second substrate 20.

The first channel ports 21 and 22, the second channel port 23, and the third channel port 24 are all cylindrical hollows extending in the Z direction. In this example, the first channel ports 21 and 22, the second channel port 23, and the third channel port 24 are all the same in diameter d (see Fig. 3), and the diameter d is, for example, 1 to 5 mm.

An interval p1 (see Fig. 3) between the first channel ports 21 and 22 is, for example, 3 to 10 mm and is 10 mm in this embodiment. An interval p2 (see Fig. 3) between the first channel port 21 and the second channel port 23 is, for example, 1 to 3 mm. An interval p3 (see Fig. 3) between the first channel port 22 and the third channel port 24 is, for example, 1 to 3 mm.

The first channel ports 21 and 22, the second channel port 23, and the third channel port 24 have at least one purpose selected from the purpose of injecting a liquid into the microfluidic device 1 and the purpose of discharging a liquid from the microfluidic device 1. For example, the first channel port 21 may be used as a liquid inlet and the first channel port 22 may be used as a liquid outlet.

The first channel port 21 and the first channel port 22 are connected through the first channel 25. That is, the first channel 25 can also be said to have the first channel ports 21 and 22 connected to the first channel 25 and extending toward the principal surface 20b. The first channel port 21 and the second channel port 23 are connected through the second channel 26. That is, the second channel 26 can also be said to have the second channel port 23 connected to the second channel 26 and extending toward the principal surface 20b. The first channel port 22 and the third channel port 24 are connected through the third channel 27. That is, the third channel 27 can also be said to have the third channel port 24 connected to the third channel 27 and extending toward the principal surface 20b.

As has been described above, the microfluidic device 1 according to this embodiment includes the first substrate 10 and the second substrate 20 partially bonded to the first substrate 10. Further, the microfluidic device 1 includes the first channel 25 extending in a direction along the principal surface 20a of the second substrate 20 between the first substrate 10 and the second substrate 20, the second channel 26 connected to the first channel 25 and extending in a direction along the principal surface 20a, and the third channel 27 connected to the first channel 25 and extending in a direction along the principal surface 20a. Further, the microfluidic device 1 includes the first channel ports 21 and 22 connected to the first channel 25 and formed to pass through the second substrate 20 toward the principal surface 20b located on the opposite side from the principal surface 20a of the second substrate 20, the at least one second channel port 23 connected to the second channel 26 and formed to pass through the second substrate 20 toward the principal surface 20b, and the at least one third channel port 24 connected to the third channel 27 and formed to pass through the second substrate 20 toward the principal surface 20b. The principal surface 20b-side wall surface 25d of the first channel 25 is closer to the principal surface 20b than the principal surface 20b-side wall surface 26d of the second channel 26 and the principal surface 20b-side wall surface 27d of the third channel 27.

Hereinbelow, a method for producing the microfluidic device 1 will be described in detail.

### (Substrate preparing step)

The first substrate 10 and the second substrate 20 to form the microfluidic device 1 are prepared.

A material used to form the substrates 10 and 20 is preferably a substantially non-porous material. The "substantially non-porous material" herein refers to a state where the apparent surface area of the substrate is approximate to the actual surface area. Examples of a material to form such a non-porous material include an inorganic material such as glass or silicon and a resin material such as polymethyl methacrylate (PMMA), polycarbonate (PC), cycloolefin copolymer (COC), cycloolefin polymer (COP), polystyrene (PS), or silicone. It should be noted that these resin materials may be used in combination of two or more of them. The material used to form the first substrate 10 and the material used to form the second substrate 20 may be different.

The shape of the substrate in this embodiment will be described. As each of the first substrate 10 and the second substrate 20, a rectangular substrate is used which has principal surfaces having the same shape. The thickness of the second substrate 20 is larger than that of the first substrate 10. However, the first substrate 10 and the second substrate 20 may be different in the shape of the principal surfaces. For example, the lengthwise dimension and widthwise dimension of principal surface of the first substrate 10 may be larger than those of principal surface of the second substrate 20 or the lengthwise dimension and the widthwise dimension of principal surface of the second substrate 20 may be larger than those of principal surface of the first substrate 10. The thickness of the second substrate 20 may be the same as that of the first substrate 10 or the thickness of the second substrate 20 may be smaller than that of the first substrate 10.

In this embodiment, the two principal surfaces 10a and 10b of the first substrate 10 are both flat. The one principal surface 20a of the second substrate 20 has the first recess 25a, the second recess 26a, and the third recess 27a for respectively forming the hollow first channel 25, the hollow second channel 26, and the hollow third channel 27 after bonding to the first substrate 10. In the other principal surface 20b of the second substrate 20, the first channel ports 21 and 22, the second channel port 23, and the third channel port 24 are opened.

In order to provide openings and recesses in the substrates 10 and 20, a means such as injection molding, a combination of a photolithographic process and an etching process, casting, or cutting work may be used, but an optimum means may be selected depending on the material forming the substrate. For example, as described above, when the second substrate 20 is formed of a resin material such as polymethyl methacrylate (PMMA), polycarbonate (PC), cycloolefin copolymer (COC), cycloolefin polymer (COP), polystyrene (PS), silicone, or acrylic, recesses can easily be formed by injection molding. When recesses are not provided in the first substrate 10, the first substrate 10 may be formed using a glass material, such as borosilicate glass, other than the resin material.

### (Substrate bonding step)

The principal surface 10a of the produced first substrate 10 and the principal surface 20a of the produced second substrate 20 are bonded together. A bonding method described below does not require formation of a thin film as an adhesive on the substrate and is performed in the following procedure.

First, the bonding surfaces (10a, 20a) of both of the substrates are subjected to surface activation treatment. As a method of the surface activation treatment, a method including irradiation with ultraviolet rays or a method including contact with plasma gas can be used.

The method including irradiation with ultraviolet rays is performed by, for example, irradiating the principal surface 20a of the second substrate 20 and the principal surface 10a of the first substrate 10 with vacuum ultraviolet rays having a wavelength of 200 nm or less emitted from an ultraviolet light source such as a xenon excimer lamp to emit light having a wavelength of 172 nm. As another example of the ultraviolet light source, a low-pressure mercury lamp having an emission line at 185 nm and a deuterium lamp having an emission line in a wavelength range of 120 to 200 nm can suitably be used. The illuminance of the vacuum ultraviolet rays is, for example, 10 to 500 mW/cm². An irradiation time is appropriately set depending on the type of resin, but is, for example, 5 to 6 seconds.

The method including contact with plasma gas is performed by generating plasma of a process gas containing nitrogen gas or argon gas as a main component and containing 0.01 to 5 vol% of oxygen gas by atmospheric-pressure plasma and bringing the plasma into contact with the principal surface 20a of the second substrate 20 and the principal surface 10a of the first substrate10. It is also possible to use a mixed gas of nitrogen gas and clean dry air (CDA). The time of contact with plasma gas is, for example, 5 to 100 seconds.

Then, a bonding step is performed in which the first substrate 10 and the second substrate 20 are stacked so that the bonding surfaces (10a, 20a) of both of the substrates subjected to surface activation treatment are in contact with each other, and both of the substrates are bonded together by pressing using a press machine. The bonding step should be performed within a predetermined time, for example, within 10 minutes after the completion of the ultraviolet irradiation step in order to maintain the surface activation state.

If necessary, the bonding step is performed in a thermal environment to achieve tight bonding. In the bonding step, bonding conditions such as heating temperature and pressing force are set depending on the constituent material of the first substrate 10 and the constituent material of the second substrate 20. As for specific conditions, the temperature during pressing is, for example, 40 to 130 °C, and the pressing force for bonding is, for example, 0.1 to 10 MPa.

If necessary, a substrate obtained by bonding the first substrate 10 and the second substrate 20 together (hereinafter sometimes referred to as a "bonded substrate") may further be heated for a predetermined time after pressurization for a predetermined time. Even when a portion achieving a satisfactory bonding state and a portion not achieving a satisfactory bonding state are mixed at a bonded interface between the stacked substrates after pressurization, a desired bonding state can be achieved in the portion not achieving a satisfactory bonding state by heating the bonded substrate after pressurization.

After the pressurization of the bonded substrate is maintained for a predetermined time and then ended, the temperature of the bonded substrate may be increased to and maintained at a predetermined temperature until a desired bonding state is achieved. Here, the predetermined temperature is a temperature at which deformation of the bonded substrate does not occur. For example, a heating temperature is, for example, 40 to 130 °C and a heating time is, for example, 60 to 600 seconds.

Then, the microfluidic device 1 in which the second substrate 20 is bonded onto the principal surface 10a of the first substrate 10 is produced through a cooling step.

Hereinbelow, a method for using the microfluidic device 1 will be described in detail. The method of use is roughly divided into two: (1) double-sided perfusion and (2) single-sided perfusion depending on the way of perfusion. Double-sided perfusion is performed by flowing a culture medium on both of upper and lower sides of a gel and is also called a two-channel perfusion (multi-channel perfusion). On the other hand, single-sided perfusion is performed by flowing a culture medium on the upper side of a gel and is also called a single-channel perfusion.

Further, the method of use is divided depending on the way of cell culture such as (a) On-Gel culture, (b) On&ln-Gel culture, or (c) In-Gel culture. In On-Gel culture, cells are cultured on the upper side of a gel. In On&ln-Gel culture, cells are cultured both on the upper side of a gel and in the gel. In In-Gel culture, cells are cultured in a gel.

There are six usage examples as combinations of them. Further, air-liquid interface culture can be performed by flowing air instead of a culture medium on a cell layer barrier.

### (1) Double-sided perfusion

### <Usage Example 1 >

### (a) On-Gel culture

For example, transmigration of white blood cells or tumor cells toward a chemoattractant through vascular endothelium can be measured.

### i) Formation of cell support

First, as shown in Fig. 6A, glycerol or thermal phase transition hydrogel 91 is injected through the second channel port 23. At this time, the amount of the glycerol or thermal phase transition hydrogel 91 to be injected is set so that the height of the injected glycerol or thermal phase transition hydrogel 91 is higher than the wall surface 26d of the second channel 26 and the wall surface 27d of the third channel 27 and lower than the wall surface 25d of the first channel 25.

Then, as shown in Fig. 6B, an aqueous solution of gel 92 is injected through the first channel port 21 to be developed on the glycerol or thermal phase transition hydrogel 91 and is then gelatinized.

Then, as shown in Fig. 6C, the glycerol or thermal phase transition hydrogel 91 is removed through the second channel port 23 or the third channel port 24. As a result, the gel 92 remains so that a space is created between a layer of the gel 92 and the first substrate 10.

### ii) Formation of cell layer barrier

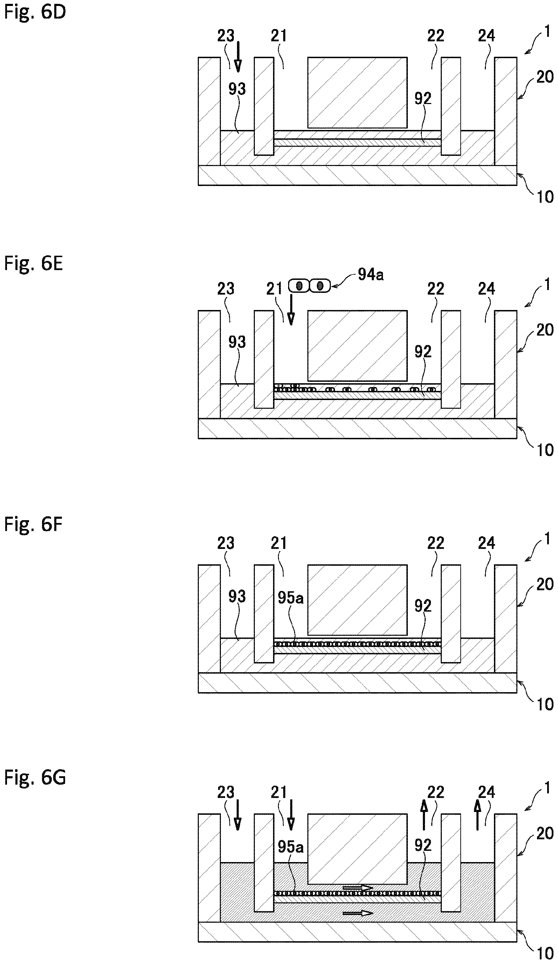

Then, as shown in Fig. 6D, a culture medium 93 is injected through the second channel port 23.

Then, as shown in Fig. 6E, the culture medium 93 containing cells 94a is injected through the first channel port 21 to culture the cells 94a. As a result, as shown in Fig. 6F, a cell layer barrier 95a is formed on the gel 92.

### iii) Perfusion of culture medium • observation

Then, as shown in Fig. 6G, a culture medium, a chemical substance, and cells are injected through the second channel port 23 and the first channel port 21 and are collected through the third channel port 24 and the first channel port 22, respectively. Specifically, action or behavior of the injected chemical substance or cells reflecting its/their migration route can biochemically and molecular biologically be analyzed by performing sampling on both sides of the cell layer barrier 95a. Further, diffusion and influence of the injected chemical substance or cells in and on a tissue model can also histologically be analyzed using a microscope or the like.

Further, a liquid can be perfused on each of both sides of the cell layer barrier 95a according to a constructed tissue model.

For example, a small intestine model is constructed by forming a cell layer barrier 95a from small intestinal endothelium.

A culture medium containing a chemical substance is perfused from the first channel port 21 to the first channel port 22 to diffuse the chemical substance from the upper side to the lower side of the constructed cell layer barrier 95a through the cell layer barrier 95a. Then, the culture medium is perfused from the second channel port 23 to the third channel port 24 and collected.

In this way, the absorption efficiency, metabolism, and toxicity of the chemical substance can be assayed by, for example, analyzing the degree of diffusion and structural change of the chemical substance and observing cells.

For example, a blood vessel model is constructed by forming a cell layer barrier 95a from vascular endothelial cells.

A culture medium containing a chemical substance is perfused from the first channel port 21 to the first channel port 22, and the culture medium containing no chemical substance is perfused from the second channel port 23 to the third channel port 24 and collected.

This makes it possible to generate a stable concentration gradient of the chemical substance in the gel 92 to assay the effect of the chemical substance on angiogenesis.

### <Usage Example 2 >

### (b) On&ln-Gel culture

The permeation, absorption, metabolism, and others of a physiological active substance, a drug, or the like through a cell barrier layer can be assayed. For example, permeability and toxicity through the cornea, absorption efficiency, metabolism, and toxicity through the intestinal tract, or permeability and toxicity through the blood vessel can be assayed.

### i) Formation of cell support

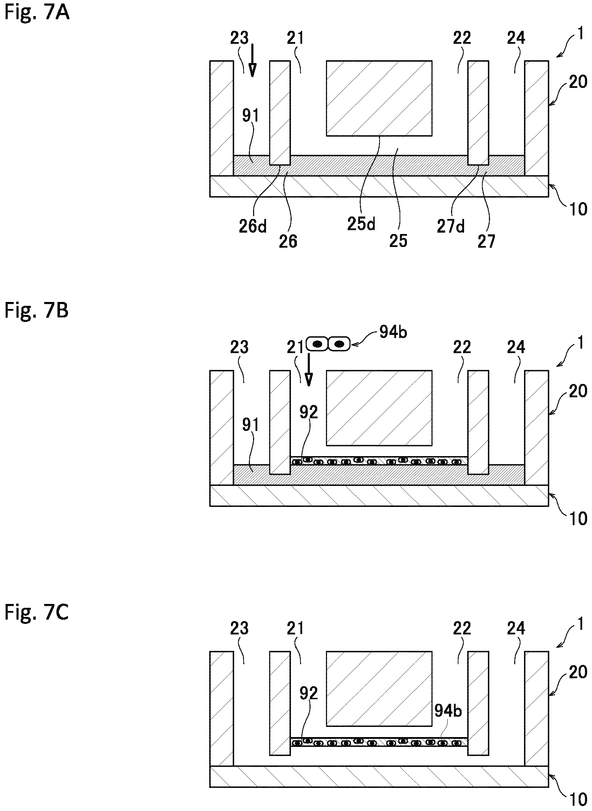

First, as shown in Fig. 7A, glycerol or thermal phase transition hydrogel 91 is injected through the second channel port 23. At this time, the amount of the glycerol or thermal phase transition hydrogel 91 to be injected is set so that the height of the injected glycerol or thermal phase transition hydrogel 91 is higher than the wall surface 26d of the second channel 26 and the wall surface 27d of the third channel 27 and lower than the wall surface 25d of the first channel 25.

Then, as shown in Fig. 7B, an aqueous solution of gel 92 containing cells 94b is injected through the first channel port 21 to be developed on the glycerol or thermal phase transition hydrogel 91 and is then gelatinized.

Then, as shown in Fig. 7C, the glycerol or thermal phase transition hydrogel 91 is removed through the second channel port 23 or the third channel port 24. As a result, the gel 92 containing the cells 94b remains so that a space is created between a layer of the gel 92 and the first substrate 10.

### ii) Formation of cell layer barrier

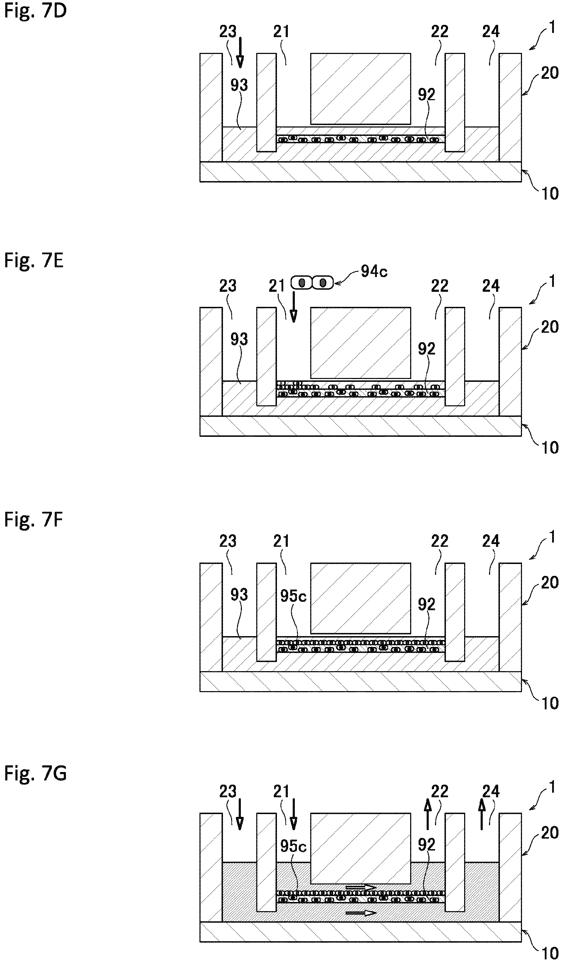

Then, as shown in Fig. 7D, a culture medium 93 is injected through the second channel port 23.

Then, as shown in Fig. 7E, the culture medium 93 containing cells 94c is injected through the first channel port 21 to culture the cells 94c. As a result, as shown in Fig. 7F, a cell layer barrier 95c is formed on the gel 92 containing the cells 94b.

### iii) Perfusion of culture medium • observation

Then, as shown in Fig. 7G, a culture medium, a chemical substance, and cells are injected through the second channel port 23 and the first channel port 21 and are collected through the third channel port 24 and the first channel port 22, respectively. Specifically, action or behavior of the injected chemical substance or cells reflecting its/their migration route can biochemically and molecular biologically be analyzed by performing sampling on both of the cell layer barrier 95c side and the cell 94b-containing gel 92 side. Further, diffusion and influence of the injected chemical substance or cells in and on a tissue model can also histologically be analyzed using a microscope or the like.

Further, a liquid can be perfused on each of both of the cell layer barrier 95c side and the cell 94b-containing gel 92 side according to a constructed tissue model.

For example, a basement membrane model is constructed by forming a cell layer barrier 95c from endothelium or epithelial cells on a gel 92 formed from extracellular matrix (ECM) in which tumor cells are embedded.

A culture medium containing a chemical substance is perfused from the first channel port 21 to the first channel port 22 to allow the chemical substance to permeate through the cell layer barrier 95c and act on the cells embedded in the extracellular matrix.

This makes it possible to assay transmigration (invasion activity) of tumor cells associated with ECM decomposition caused by the chemical substance permeated through the cell layer barrier 95c.

For example, a blood vessel model is constructed by forming a cell layer barrier 95c from vascular endothelium on a gel 92 formed from extracellular matrix (ECM) in which fibroblast cells or tumor cells are embedded.

A culture medium containing a chemical substance is perfused from the first channel port 21 to the first channel port 22 to allow the chemical substance to permeate through the cell layer barrier 95c and act on the cells embedded in the extracellular matrix to produce cytokine.

This makes it possible to assay the effect of cytokine on angiogenesis.

### <Usage Example 3>

### (c) In-Gel culture

For example, transmigration of cells such as fibroblast cells or tumor cells toward a chemoattractant (chemical signal) in an environment around the cells can be measured.

### i) Formation of cell support

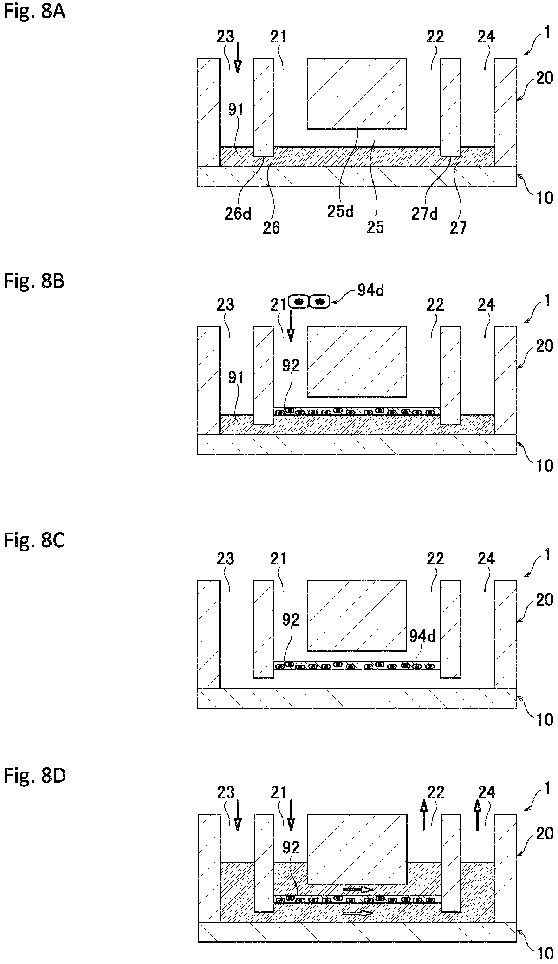

First, as shown in Fig. 8A, glycerol or thermal phase transition hydrogel 91 is injected through the second channel port 23. At this time, the amount of the glycerol or thermal phase transition hydrogel 91 to be injected is set so that the height of the injected glycerol or thermal phase transition hydrogel 91 is higher than the wall surface 26d of the second channel 26 and the wall surface 27d of the third channel 27 and lower than the wall surface 25d of the first channel 25.

Then, as shown in Fig. 8B, an aqueous solution of gel 92 containing cells 94d is injected through the first channel port 21 to be developed on the glycerol or thermal phase transition hydrogel 91 and is then gelatinized.

Then, as shown in Fig. 8C, the glycerol or thermal phase transition hydrogel 91 is removed through the second channel port 23 or the third channel port 24. As a result, the gel 92 containing the cells 94d remains so that a space is created between a layer of the gel 92 and the first substrate 10.

### ii) Perfusion of culture medium • observation

Then, as shown in Fig. 8D, a culture medium, a chemical substance and cells are injected through the second channel port 23 and the first channel port 21 and are collected through the third channel port 24 and the first channel port 22, respectively. Specifically, action or behavior of the injected chemical substance or cells reflecting its/their migration route can biochemically and molecular biologically be analyzed by performing sampling on both sides of the gel 92 containing the cells 94d. Further, diffusion and influence of the injected chemical substance or cells in and on a tissue model can also histologically be analyzed using a microscope or the like.

Further, a liquid can be perfused on each of both sides of the gel 92 containing the cells 94d according to a constructed tissue model.

For example, fibroblast cells, tumor cells, or the like are embedded in the gel 92. A culture medium containing a chemoattractant is perfused from the first channel port 21 to the first channel port 22, and the culture medium containing no chemoattractant is perfused from the second channel port 23 to the third channel port 24 and collected.

This makes it possible to generate a stable concentration gradient of the chemoattractant in the gel 92 to assay cell transmigration toward the chemoattractant.

As has been described above, a cell layer barrier can be formed without an artificial membrane in double-sided perfusion culture.

### (2) Single-sided perfusion

### <Usage Example 4>

### (a) On-Gel culture

For example, transmigration of white blood cells or tumor cells toward a chemoattractant through vascular endothelium can be measured.

### i) Formation of cell support

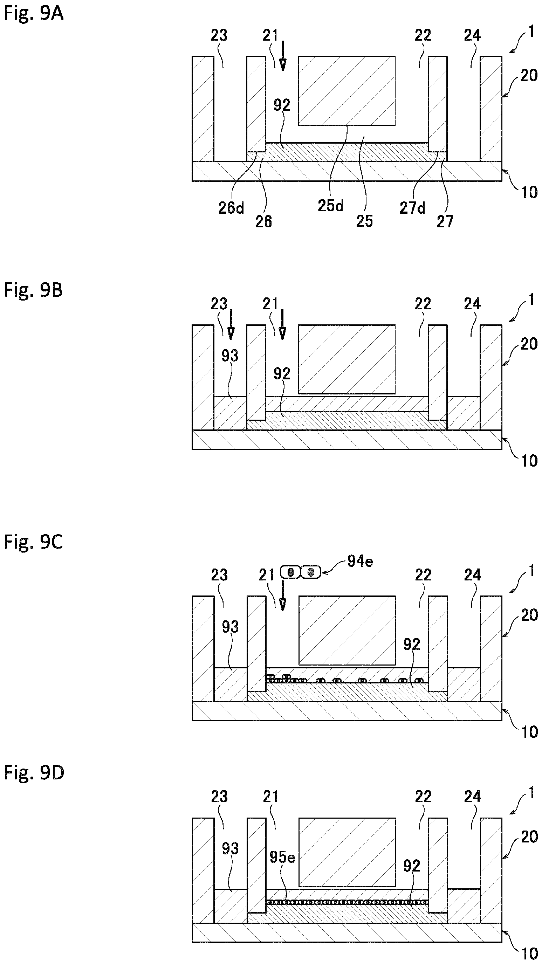

First, as shown in Fig. 9A, an aqueous solution of gel 92 is injected through the first channel port 21 and gelatinized. The injected gel 92 stays almost in a range between the first channel ports 21 and 22 due to capillary force and surface tension. At this time, the amount of the gel 92 to be injected is set so that the height of the injected gel 92 is higher than the wall surface 26d of the second channel 26 and the wall surface 27d of the third channel 27 and lower than the wall surface 25d of the first channel 25.

### ii) Formation of cell layer barrier

Then, as shown in Fig. 9B, a culture medium 93 is injected through the second channel port 23 and/or the first channel port 21.

Then, as shown in Fig. 9C, cells 94e are injected through the first channel port 21 and cultured. As a result, as shown in Fig. 9D, a cell layer barrier 95e is formed on the gel 92.

### iii) Perfusion of culture medium • observation

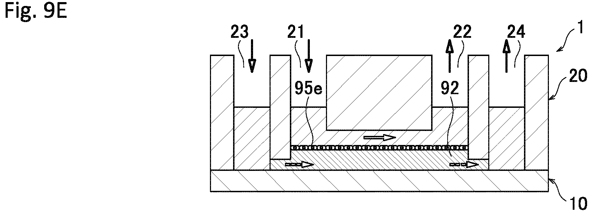

Then, as shown in Fig. 9E, a culture medium, a chemical substance, and cells are injected through the second channel port 23 and the first channel port 21 and are collected through the third channel port 24 and the first channel port 22, respectively. Specifically, action or behavior of the injected chemical substance or cells reflecting its/their migration route can biochemically and molecular biologically be analyzed by performing sampling on both of the Apical side of the cell layer barrier 95e (in Fig. 9E, the upper side of the cell layer barrier 95e) and the Basal side of the cell layer barrier 95e (in Fig. 9E, the lower side of the cell layer barrier 95e). Further, diffusion and influence of the injected chemical substance or cells in and on a tissue model can also histologically be analyzed using a microscope or the like.

Further, a liquid can be perfused on the Apical side of the cell layer barrier 95e according to a constructed tissue model.

For example, a blood vessel model is constructed by forming a cell layer barrier 95e from vascular endothelial cells.

A culture medium containing a chemoattractant (e.g., TNα) is perfused from the first channel port 21 to the first channel port 22 to activate vascular endothelium. Then, white blood cells or tumor cells are injected through the first channel port 21 (or the first channel port 22).

This makes it possible to assay adhesion of the injected cells onto the cell layer barrier 95e or transmigration of the injected cells from the cell layer barrier 95e.

### <Usage Example 5>

### (b) On&ln-Gel culture

The permeation, absorption, metabolism, and others of a physiological active substance, a drug, or the like through a cell barrier layer can be assayed. For example, permeability and toxicity through the cornea, absorption efficiency, metabolism, and toxicity through the intestinal tract, or permeability and toxicity through the blood vessel can be assayed.

### i) Formation of cell support

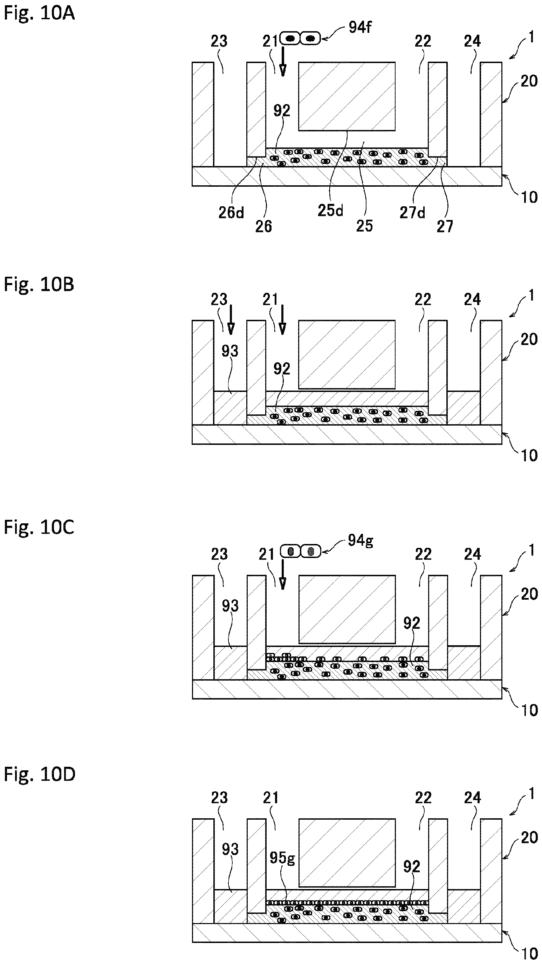

First, as shown in Fig. 10A, an aqueous solution of gel 92 containing cells 94f is injected through the first channel port 21 and gelatinized. The injected gel 92 stays almost in a range between the first channel ports 21 and 22 due to capillary force and surface tension. At this time, the amount of the gel 92 to be injected is set so that the height of the injected gel 92 is higher than the wall surface 26d of the second channel 26 and the wall surface 27d of the third channel 27 and lower than the wall surface 25d of the first channel 25.

### ii) Formation of cell layer barrier

Then, as shown in Fig. 10B, a culture medium 93 is injected through the second channel port 23 and/or the first channel port 21.

Then, as shown in Fig. 10C, cells 94g are injected through the first channel port 21 and cultured. As a result, as shown in Fig. 10D, a cell layer barrier 95g is formed on the gel 92.

### iii) Perfusion of culture medium • observation

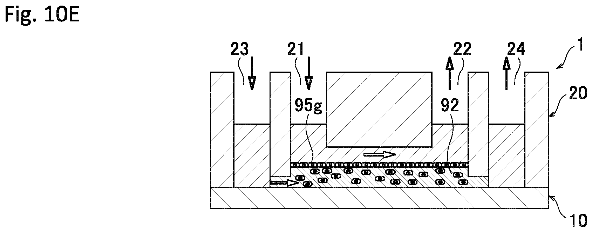

Then, as shown in Fig. 10E, a culture medium, a chemical substance, and cells are injected through the second channel port 23 and the first channel port 21 and are collected through the third channel port 24 and the first channel port 22, respectively. Specifically, action or behavior of the injected chemical substance or cells reflecting its/their migration route can biochemically and molecular biologically be analyzed by performing sampling on both of the cell layer barrier 95g side and the cell 94f-containing gel 92 side. Further, diffusion and influence of the injected chemical substance or cells in and on a tissue model can also histologically be analyzed using a microscope or the like.

Further, a liquid can be perfused on the Apical side of the cell layer barrier 95g according to a constructed tissue model.

It should be noted that in this example, a basement membrane model or a blood vessel model can be constructed and assayed as in the case of the example of double-sided perfusion and On&ln-Gel culture.

### <Usage Example 6>

### (c) In-Gel culture

For example, transmigration of cells such as fibroblast cells or tumor cells toward a chemoattractant (chemical signal) in an environment around the cells can be measured.

### i) Formation of cell support

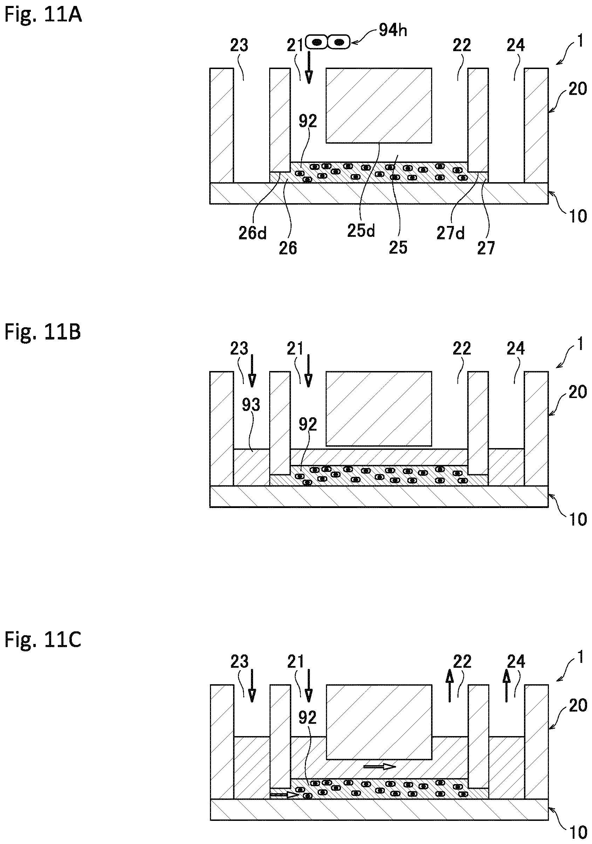

First, as shown in Fig. 11A, an aqueous solution of gel 92 containing cells 94h is injected through the first channel port 21 and gelatinized. The injected gel 92 stays almost in a range between the first channel ports 21 and 22 due to capillary force and surface tension. At this time, the amount of the gel 92 to be injected is set so that the height of the injected gel 92 is higher than the wall surface 26d of the second channel 26 and the wall surface 27d of the third channel 27 and lower than the wall surface 25d of the first channel 25.

Then, as shown in Fig. 11B, a culture medium 93 is injected through the second channel port 23 and/or the first channel port 21.

### ii) Perfusion of culture medium • observation

Then, as shown in Fig. 11C, a culture medium, a chemical substance, and cells are injected through the second channel port 23 and the first channel port 21 and are collected through the third channel port 24 and the first channel port 22, respectively. Specifically, action or behavior of the injected chemical substance or cells reflecting its/their migration route can biochemically and molecular biologically be analyzed by performing sampling on both sides of the gel 92 containing the cells 94h. Further, diffusion and influence of the injected chemical substance or cells in and on a tissue model can also histologically be analyzed using a microscope or the like.

Further, a liquid can be perfused on the upper side of the gel 92 containing the cells 94h according to a constructed tissue model.

As has been described above, a cell layer barrier can be formed without an artificial membrane in single-sided perfusion culture.

### (3) Air-liquid interface culture

Absorption efficiency and toxicity through skin can be assayed by forming keratinocyte or fibroblast cells as a cell barrier layer.

### < Usage Example 7>

### i) Formation of cell support

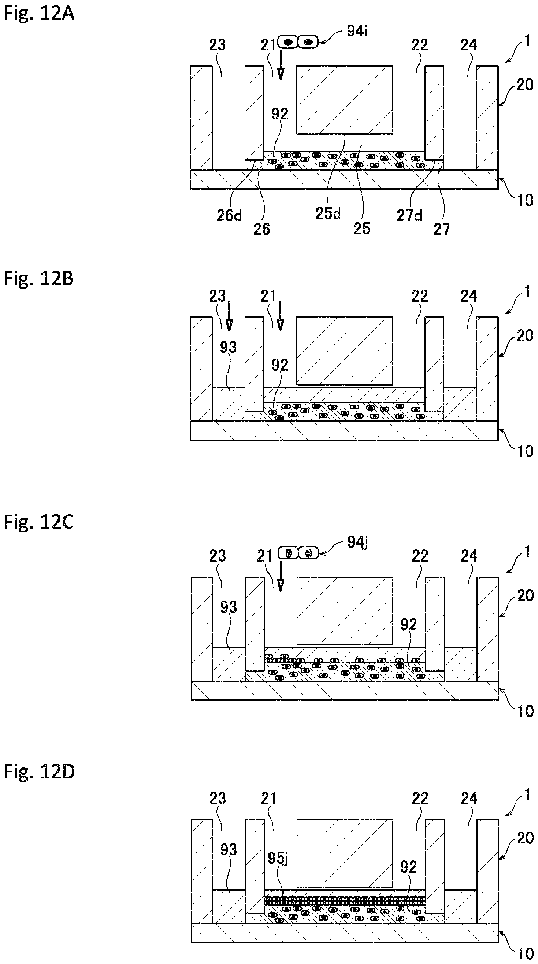

First, as shown in Fig. 12A, an aqueous solution of gel 92 containing cells 94i is injected through the first channel port 21 and gelatinized. The injected gel 92 stays almost in a range between the first channel ports 21 and 22 due to capillary force and surface tension. At this time, the amount of the gel 92 to be injected is set so that the height of the injected gel 92 is higher than the wall surface 26d of the second channel 26 and the wall surface 27d of the third channel 27 and lower than the wall surface 25d of the first channel 25.

### ii) Formation of cell layer barrier

Then, as shown in Fig. 12B, a culture medium 93 is injected through the second channel port 23 and/or the first channel port 21.

Then, as shown in Fig. 12C, cells 94j are injected through the first channel port 21 and cultured. As a result, as shown in Fig. 12D, a cell layer barrier 95j is formed on the gel 92.

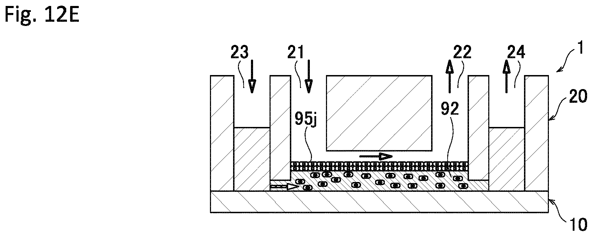

Then, as shown in Fig. 12E, the culture medium on the cell layer barrier 95j side is removed to expose the cell layer barrier 95j to air. When keratinocyte is cultured as the cell layer barrier 95j, growth and differentiation into stratified squamous epithelium by stratification of keratinocyte can be expressed.

As has been described above, the method for using the microfluidic device 1 according to this embodiment may include a first step in which glycerol or thermal phase transition hydrogel 91 is injected through the second channel port 23 or the third channel port 24 so that a height of the glycerol or thermal phase transition hydrogel 91 is higher than the principal surface 20b-side wall surface 26d of the second channel 26 and the principal surface 20b-side wall surface 27d of the third channel 27 and lower than the principal surface 20b-side wall surface 25d of the first channel 25; a second step in which an aqueous solution of gel 92 is injected through the first channel port 21; a third step in which the aqueous solution of the gel 92 is gelatinized; and a fourth step in which the glycerol or thermal phase transition hydrogel 91 is discharged through the third channel port 24 or the second channel port 23.

A material as a base for the gel 92 is required to have a higher specific gravity than the gel 92. This is because if the gel 92 has a higher specific gravity, the gel 92 sinks into the base for the gel 92 and therefore a layer of the gel 92 cannot be formed. As a specific example of such a material, glycerol or thermal phase transition hydrogel can be used. The thermal phase transition hydrogel is preferably liquidized at 4 to 37°C.

The gel 92 that can be used is required to be gelatinized under physiological conditions (37 °C) and have no cell toxicity. Specific examples thereof include naturally-occurring polymer gel and synthetic polymer gel.

Examples of the naturally-occurring polymer gel include collagen (type I, II, III, V, or XI), a reconstituted basement membrane extracted from EHS mouse tumor (containing collagen type IV, laminin, heparan sulfate proteoglycan, and others) (trade name: Matrigel), gelatin, agar, agarose, fibrin, glycosaminoglycan, hyaluronic acid, and proteoglycan.

Examples of the synthetic polymer gel include polyacrylamide, polyvinyl alcohol, methyl cellulose, polyethylene oxide, poly(ll-hydroxyethyl methacrylate)/polycaprolactone, and a mixture of two or more of them.

As has been described above, a cell layer barrier can be formed without using an artificial membrane in air-liquid interface culture.

Although the embodiments according to the present invention have been described above with reference to the drawings, it should be understood that specific configurations are not limited to these embodiments. The scope of the present invention is indicated not only by the above description of the embodiments but also by the claims, and all changes which come within the meaning and range of equivalency of the claims are therefore intended to be embraced therein.

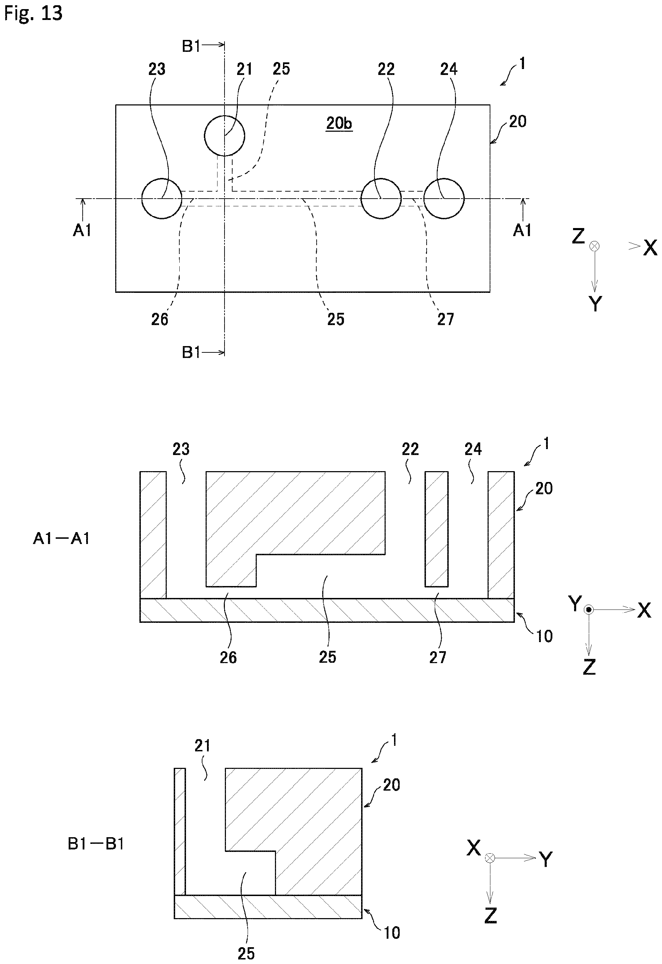

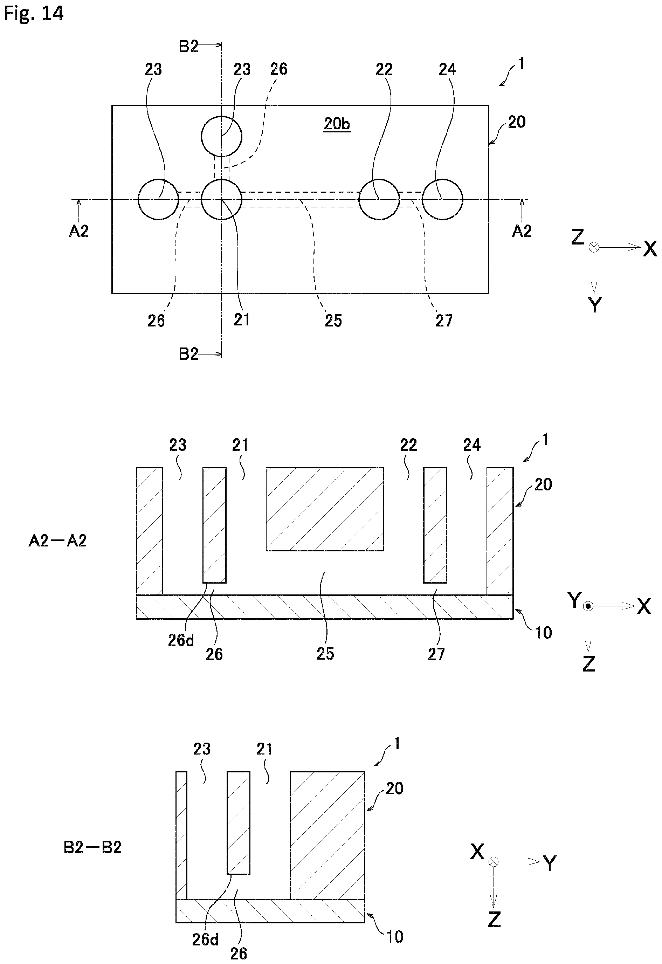

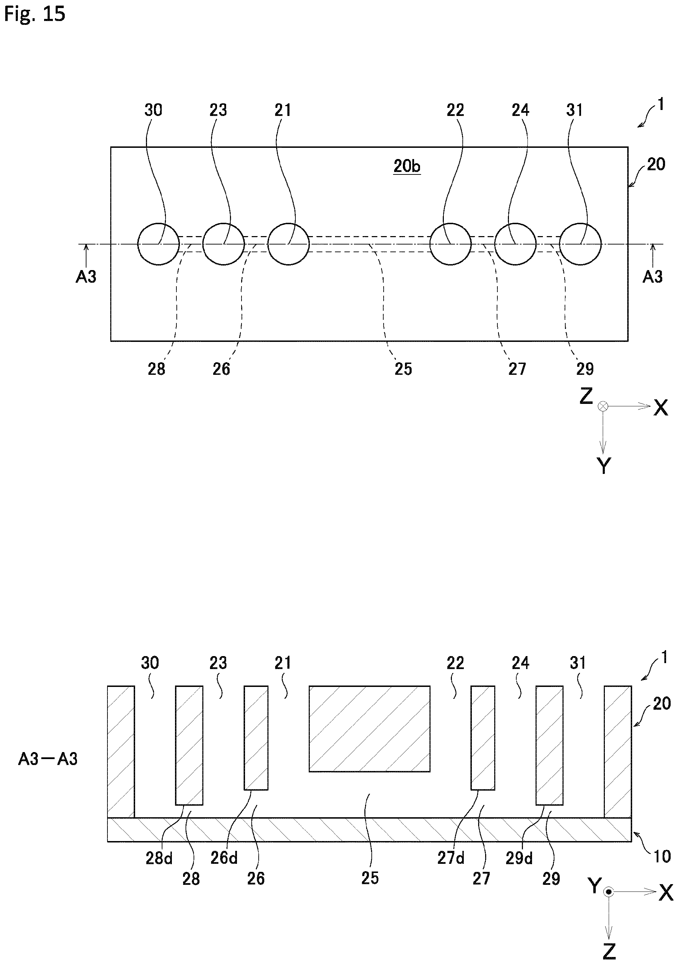

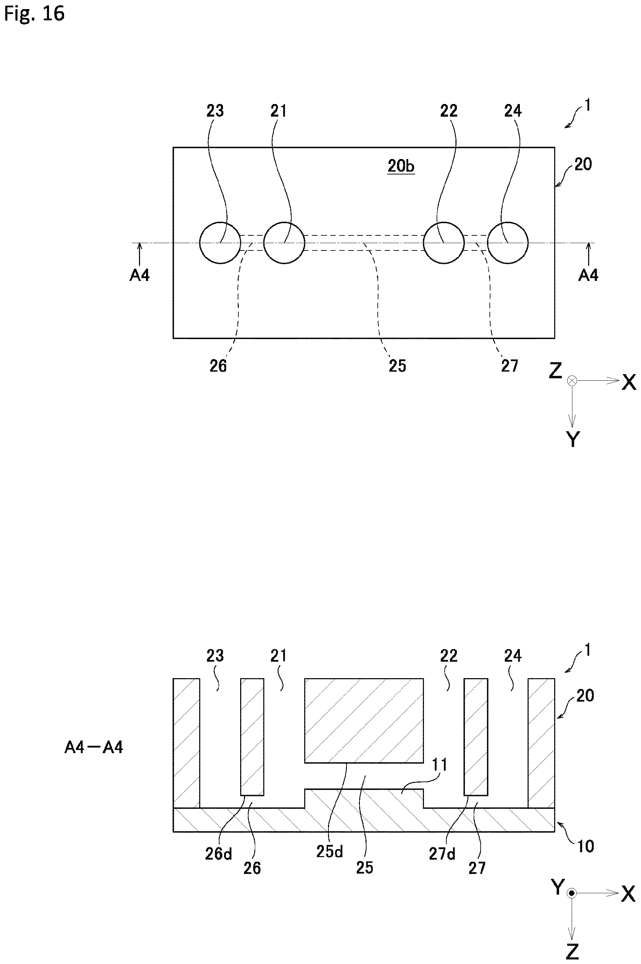

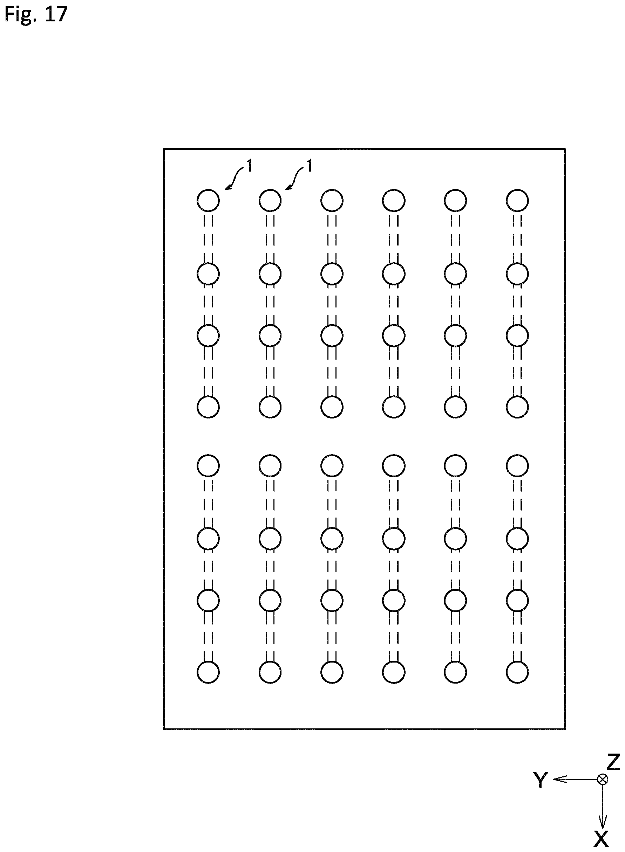

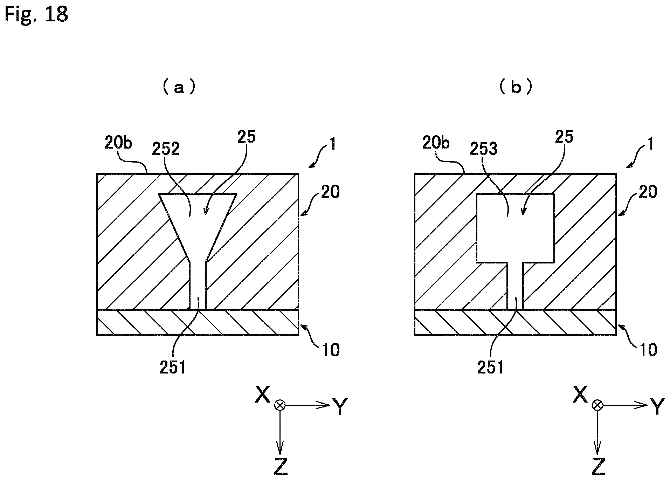

The structure adopted in each of the above embodiments can be adopted in any other embodiment. Specific configurations of parts are not limited only to those in the above-described embodiments, and various modifications can be made without departing from the spirit of the present invention.
(1) In the microfluidic device 1 according to the above embodiment, the second channel 26 is connected to the end of the first channel 25 but may be connected to the middle of the first channel 25. In an example shown in Fig. 13, the first channel 25 is formed in an L shape, and the second channel 26 is connected to the bend of the first channel 25.
(2) The second channel 26 may include a plurality of channels. In an example shown in Fig. 14, two second channels 26 are connected to the first channel 25. It should be noted that in this example, the two second channels 26 are different in the height of the wall surface 26d, but may be the same in the height of the wall surface 26d.
(3) As shown in Fig. 15, the microfluidic device 1 may include a fourth channel 28 connected to the second channel 26 and extending in a direction along the principal surface 20a, a fifth channel 29 connected to the third channel 27 and extending in a direction along the principal surface 20a, at least one fourth channel port 30 connected to the fourth channel 28 and formed to pass through the second substrate 20 toward the principal surface 20b, and at least one fifth channel port 31 connected to the fifth channel 29 and formed to pass through the second substrate 20 toward the principal surface 20b, wherein a principal surface 20b-side wall surface 28d of the fourth channel 28 and a principal surface 20b-side wall surface 29d of the fifth channel 29 are closer to the principal surface 20a than the principal surface 20b-side wall surface 26d of the second channel 26 and the principal surface 20b-side wall surface 27d of the third channel 27.
(4) In the microfluidic device 1 according to the above embodiment, the channels (the first channel 25, the second channel 26, and the third channel 27) are constituted from only the recesses (the first recess 25a, the second recess 26a, and the third recess 27a) formed in the principal surface 20a of the second substrate 20, but the channels are not limited thereto. For example, as shown in Fig. 16, the first channel 25 may be constituted from a projection 11 formed on the principal surface 10aof the first substrate 10 and the first recess 25a of the second substrate 20. Also in this case, the principal surface 20b-side wall surface 25d of the first channel 25 is closer to the principal surface 20b than the principal surface 20b-side wall surface 26d of the second channel 26 and the principal surface 20b-side wall surface 27d of the third channel 27.
(5) As shown in Fig. 17, the two or more microfluidic devices 1 may be formed in one plate.
(6) In the microfluidic device 1 according to the above embodiment, the width of the first channel 25 is constant in the Z direction, but the width of the first channel 25 is not limited thereto. As shown in Fig. 18, the width of the first channel 25 may be increased toward the second principal surface 20b. This makes it possible to reduce capillary force generated in the first channel 25. Fig. 18 corresponds to the C-C line sectional view shown in Fig. 5. In an example shown in Fig. 18(a), the first channel 25 has a constant-width portion 251 having a constant width in the Z direction and a widened portion 252 whose width is gradually increased from the width of the constant-width portion 251 toward the second principal surface 20b. In an example shown in Fig. 18(b), the first channel 25 has a constant-width portion 251 having a constant width in the Z direction and a widened portion 253 whose width is larger than the width of the constant-width portion 251 and constant in the Z direction.

### DESCRIPTION OF REFERENCE SIGNS

- 1: Microfluidic device
- 10: First substrate
- 20: Second substrate
- 20a: Principal surface
- 20b: Principal surface
- 21: First channel port
- 22: First channel port
- 23: Second channel port
- 24: Third channel port
- 25: First channel
- 25a: First recess
- 25d: Wall surface
- 26: Second channel
- 26a: Second recess
- 26d: Wall surface
- 27: Third channel
- 27a: Third recess
- 27d: Wall surface
- 28: Fourth channel
- 28d: Wall surface
- 29: Fifth channel
- 29d: Wall surface
- 30: Fourth channel port
- 31: Fifth channel port
- 91: Glycerol or thermal phase transition hydrogel
- 92: Gel

## Claims

1. A microfluidic device comprising:
a first substrate;
a second substrate partially bonded to the first substrate;
a first channel extending in a direction along a first principal surface of the second substrate between the first substrate and the second substrate;
a second channel connected to the first channel and extending in a direction along the first principal surface;
a third channel connected to the first channel and extending in a direction along the first principal surface;
a plurality of first channel ports connected to the first channel and formed to pass through the second substrate toward a second principal surface located opposite to the first principal surface of the second substrate;
at least one second channel port connected to the second channel and formed to pass through the second substrate toward the second principal surface; and
at least one third channel port connected to the third channel and formed to pass through the second substrate toward the second principal surface,
wherein a second principal surface-side wall surface of the first channel is closer to the second principal surface than a second principal surface-side wall surface of the second channel and a second principal surface-side wall surface of the third channel.

2. The microfluidic device according to claim 1, wherein the second channel is connected to a middle of the first channel.

3. The microfluidic device according to claim 1, wherein the second channel includes a plurality of second channels.

4. The microfluidic device according to claim 1, comprising a fourth channel connected to the second channel and extending in a direction along the first principal surface, a fifth channel connected to the third channel and extending in a direction along the first principal surface, at least one fourth channel port connected to the fourth channel and formed to pass through the second substrate toward the second principal surface, and at least one fifth channel port connected to the fifth channel and formed to pass through the second substrate toward the second principal surface,
wherein a second principal surface-side wall surface of the fourth channel and a second principal surface-side wall surface of the fifth channel are closer to the first principal surface than the second principal surface-side wall surface of the second channel and the second principal surface-side wall surface of the third channel.

5. The microfluidic device according to claim 1, wherein a width of the first channel is increased toward the second principal surface.

6. A method for using the microfluidic device according to claim 1, comprising:
a first step in which glycerol or thermal phase transition hydrogel is injected through the second channel port or the third channel port so that a height of the glycerol or the thermal phase transition hydrogel is higher than the second principal surface-side wall surface of the second channel and the second principal surface-side wall surface of the third channel and lower than the second principal surface-side wall surface of the first channel;
a second step in which an aqueous solution of gel is injected through one of the first channel ports;
a third step in which the aqueous solution of gel is gelatinized; and
a fourth step in which the glycerol or the thermal phase transition hydrogel is discharged through the third channel port or the second channel port.
